**Europäisches Patentamt**

**European Patent Office**

**Office européen des brevets**

(11) Veröffentlichungsnummer: **0 419 878 B1**

(12) **EUROPÄISCHE PATENTSCHRIFT**

(45) Veröffentlichungstag der Patentschrift: **19.10.94**

(21) Anmeldenummer: **90116631.4**

(22) Anmeldetag: **30.08.90**

(51) Int. Cl.⁵: **C12N 15/15**, C12P 21/02, A61K 37/64

(54) Neue Aprotinin-Varianten und gentechnisches Verfahren zur mikrobiellen Herstellung dieser Varianten in homogen prozessierter Form.

(30) Priorität: **13.09.89 DE 3930522**

(43) Veröffentlichungstag der Anmeldung:
**03.04.91 Patentblatt 91/14**

(45) Bekanntmachung des Hinweises auf die
Patenterteilung:
**19.10.94 Patentblatt 94/42**

(84) Benannte Vertragsstaaten:
**AT BE CH DE DK ES FR GB GR IT LI LU NL SE**

(56) Entgegenhaltungen:
**EP-A- 0 307 592**
**EP-A- 0 339 942**
**WO-A-89/01968**

(73) Patentinhaber: **BAYER AG**

**D-51368 Leverkusen (DE)**

(72) Erfinder: **Ebbers, Jürgen, Dr.**
**Paul-Ehrlich Strasse 10**
**D-5600 Wuppertal 1 (DE)**
Erfinder: **Hörlein, Dietrich, Dr.**
**Paul-Ehrlich Strasse 20**
**D-5600 Wuppertal 1 (DE)**
Erfinder: **Schedel, Michael, Dr.**
**Gellertweg 37**
**D-5600 Wuppertal 1 (DE)**
Erfinder: **Das, Rathindra, Dr.**
**Pahlkestrasse 15**
**D-5600 Wuppertal 1 (DE)**

**Beschreibung**

Die vorliegende Erfindung betrifft rekombinante Aprotinin-Varianten; Verfahren, die beschriebenen Peptidvarianten als homogen prozessierte Sekretionsprodukte mit transformierten Hefen herzustellen sowie die rekombinanten Aprotinin-Varianten enthaltende Arzneimittel.

Aprotinin ist ein gut untersuchtes Protein mit 58 Aminosäuren, welches inhibitorisch auf Trypsin, Chymotrypsin, Plasmin und Plasma-Kallikrein wirkt (H. Fritz und G. Wunderer, Drug. Res. 33, 479 - 494, 1983; W. Gebhard, H. Tschesche und H. Fritz, Proteinase Inhibitors, Barrett und Salvesen (eds.), Elsevier Science Publ. BV 375 - 387, 1986).

Das aus Organen des Rindes gewonnene Aprotinin ist die Wirksubstanz das Arzneimittels Trasylol®, welches für die Behandlung verschiedener Erkrankungen, wie z.B. hyperfibrinolytischer Blutungen und traumat.-haemorhagischer Schock verwendet wird.

Darüber hinaus existieren neue klinische Befunde, die zeigen, daß durch die Anwendung von Trasylol® bei Operationen am offenen Herzen der durch Fibrinolyse und Koagulation bedingte Blutverlust erheblich reduziert werden kann (W. van Oeveren et al. Ann. Thorac. Surg. 44, 640 - 645, 1987; D. Royston et al. Lancet II, 1289 - 1291, 1987; B.P. Bistrup et al, Lancet I, 366 - 367, 1988).

Es konnte gezeigt werden, daß semisynthetisch erzeugte Homologe von Aprotinin, die in Pos. 15 der Aminosäuresequenz anstelle von Lysin andere Aminosäuren enthalten, ein von Aprotinin deutlich abweichendes Wirkprofil und Wirkungsspezifität besitzen (Tschesche et al., US-Patent 4 595 674; H. R. Wenzel et al. in Chemistry of Peptides and Proteins, Band 3, 1985).

Einige dieser semisynthetischen Aprotinin-Homologe besitzen beispielsweise eine stark inhibierende Wirkung auf Elastase aus Pankreas und Leukozyten. Bedingt durch diese neuartige Wirkungsspezifität können diese Aprotinin-Homologe therapeutisch bei Erkrankungen verwendet werden, die durch eine erhöhte Freisetzung von Elastase mit verursacht werden, wie z.B. Emphysem-Bildung, ARDS (Adult Respiratory Distress Syndrom) und rheumatoider Arthritis.

Andere Aprotinin-Homolge mit Arginin in Pos. 15 sind durch eine gegenüber Aprotinin deutlich gesteigerte Inhibitor-Wirkung auf das bei der Blutgerinnungskaskade zentral beteiligte Plasma-Kallikrein gekennzeichnet.

Die durch halbsynthetische Umwandlung des Rinder-Aprotinins erzielbaren Ausbeuten fallen erfahrungsgemäß gering aus. Es war daher für die Herstellung großer Mengen von Aprotinin-Homologen vorteilhaft, unter Verwendung synthetisch hergestellter Gene Gen-Produkte in prokaryotischen Mikroorganismen fermentativ herzustellen (Auerswald et al., Patent EP 01238993 A2; B v. Wilcken-Bergmann et al., EMBO J. 5, 3219 - 3225, 1986).

Für die Herstellung von rek. Aprotinin-Varianten in E. coli-K 12-Stämmen wurden beispielsweise Expressionssysteme verwendet, die das Aprotinin-Mutein als intrazellulär gebildetes Fusionsprotein mit einem geeigneten Fusionspartner, wie z.B. dem N-terminalen Peptid-Anteil der MS 2-Replikase in Form von intrazellulären Einschlußkörpern akkumulieren (E. A. Auerswald et al., Biol. Chem. Hoppe Seyler 369, 27 - 35, 1988).

Daneben können auch E. coli-Expressions-/Sekretions-Systeme verwendet werden, die durch Fusion des Aprotinin-Muteins mit geeigneten Gen-Sequenzen für sekretorische Signalpeptide, wie z.B. der OmpA-Signalsequenz oder der phoA-Signalsequenz die Sekretion von hemmaktiven Aprotinin-Varianten ins Periplasma der Bakterienzelle ermöglichen (persönliche Mitteilung, Dr. W. Bruns - Bayer AG; C.B. Marks et al., J. Biol. Chem. 261, 7115 - 7118, 1986).

Unter den eukaryotischen Systemen eignen sich besonders Hefe-Expressionssysteme für die gentechnische Herstellung von rek. Aprotinin-Varianten, bei denen das Gen-Produkt entweder intrazellulär akkumuliert wird oder als Fusion mit einer geeigneten sekretorischen Signalsequenz aus Hefen durch den Sekretionsweg geschleust und nach Abspaltung durch eine Membranprotease als hemmaktive Substanz in das Kulturmedium exportiert wird. Als geeignete Signalsequenzen für die Sekretion können beispielsweise verwendet werden die Signalsequenzen des alpha-mating-Faktors, der alpha-Amylase, der Glucoamylase oder der Ivertase.

Neben E. coli und Hefen können aber auch zahlreiche andere pro- und eukaryotische Expressions-/Sekretions-Systeme für die Herstellung von rek. Aprotinin-Varianten verwendet werden, wie z.B. Bacilli, Staphylococci oder Aspergilli.

Wie die o.a. Beispiele zeigen, ist die Expression von Aprotinin-Muteinen in verschiedenen pro- und eukaryontischen Systemen Stand der Technik.

Im Hinblick auf eine großtechnische Herstellung ist es dabei vorteilhaft, die Aprotinin-Varianten nicht in einer intrazellulär akkumulierten Form, sondern durch Nutzung der systemeigenen Sekretionsmechanismen als aktive Substanz ins Fermentationsmedium exportiert zu erhalten.

Bei der Verwendung von Hefe-Systemen werden dazu gentechnisch an den N-Terminus der Aprotinin-Varianten Signalsequenzen von sekretorischen Hefe-Proteinen, wie z.B. der alpha-Amylase, der Glucoamylase, der Invertase oder des alpha-mating-Faktors fusioniert.

Die enzymatische Abspaltung des Signalpeptids vom N-Terminus der Aprotinin-Varianten erfolgt beim Membrantransfer durch ein Hefe-eigenes Enzym, das eine spezifische Spaltsequenz (am C-Terminus des Signalpeptids) erkennt (s. Review-Artikel R. C. Das and J. L. Shultz, Biotechn. Progress 3, 43 - 48, 1987).

Im Falle der in Hefen exprimierten rek. Aprotinin-Varianten mit der natürlichen N-terminalen Aminosäuresequenz "Arg-Pro-Asp" hat sich jedoch gezeigt, daß durch eine nicht korrekte Abspaltung der o.a. Signalpeptide das sekretierte Material variable N-terminale Additionen von Aminosäure der verschiedenen fusionierten Signalpeptide aufweist. Ein einheitlich und korrekt prozessiertes, für die Aufreinigung geeignetes Sekretionsprodukt tritt nicht auf.

Die teilweise oder völlige Fehlprozessierung von Sekretionsprodukten ist in der Literatur auch für andere heterologe Proteine beschrieben, die als Fusionsprodukte mit Hefe-eigenen sekretorischen Signalsequenzen exprimiert werden (R. C. Das and J. L. Shultz, Biotechn. Progress 3, 43 - 48, 1987; P. J. Barr et al., J. Biol. Chem. 263, 16471 - 16478, 1988).

Überraschenderweise wurde nun gefunden, daß gentechnisch veränderte Aprotinin-Varianten, die beispielsweise eine Deletion der Aminosäure Prolin in Pos. 2 oder die eine N-terminale Addition von Ala-(-2)-Gln-(-1) aufweisen, in Hefe zu einem hohen Prozentsatz korrekt prozessiert werden.

Die vorliegende Erfindung betrifft also rek. Aprotinin bzw. Aprotinin-Varianten mit einer Deletion der Aminosäure Prolin in Position 2 oder einer Addition von Alanin-(-2)-Glutamin-(-1). Das Aprotinin kann humanen oder tierischen Ursprungs sein.

Ganz besonders bevorzugt sind Aprotinin-Varianten aus der Gruppe
Des Pro2-Val-15-Leu-17,
Des Pro2-Val-15-Leu-17-Arg-19,
Ala(-2)-Gln(-1)-Val15-Leu-17,
Ala(-2)-Gln(-1)-Val15-Leu17-Arg-19,
Des Pro2-Arg-15,
Des Pro2-Arg-15-Ala-17,
Ala(-2)-Gln(-1)-Arg-15,
Ala(-2)-Gln(-1)-Arg-15-Ala-17.

Weiterhin betrifft die vorliegende Erfindung Arzneimittel enthaltend eines oder mehrere der oben beschriebbenen Aprotinine.

Bevorzugt sind Aprotinine die zusätzlich zu den oben genannten Austauschen weitere Austausche in einer oder mehreren der Positionen 12, 13, 14, 15, 16, 17, 18, 19, 34, 36, 37, 38, 39, 41, 42 und 52 äufweisen. Näheres über die Austausche und die Aminosäuren die in den jeweiligen Positionen stehen können ist den Europäischen Patentanmeldungen EP 238 993, 297 362 bzw. 307 592 zu entnehmen.

In den nachfolgenden Beispielen wird die gentechnische Konstruktion von rek. Aprotinin-Muteinen beschrieben, die gegenüber dem natürlichen Aprotinin veränderte N-terminale Aminosäuresequenzen aufweisen. Desweiteren wird beispielhaft die Expression dieser Aprotinin-Muteine in Hefe als Fusionsprodukt mit der alpha-mating-Faktor-Prä-Pro-Sequenz sowie die Aufreinigung der prozessierten Sekretionsprodukte, beschrieben. Die überwiegend einheitliche N-terminale Prozessierung der isolierten Aprotinin-Varianten und deren Hemmeigenschaften werden ebenfalls gezeigt.

Methoden:

Enzyme und Standardtechniken

Die Enzyme für molekulargenetische Experimente wurden von Boehringer Mannheim (BRD), Gibco-BRL (USA) und Pharmacia (Schweden) bezogen.

Die Standardtechniken für molekulargenetische Experimente, wie z.B. Isolierung von Plasmid-DNA aus E. coli, Isolierung und Ligierung von DNA-Fragmenten für Klonierungsexperimente mit verschiedenen Enzymen sind beschrieben in Maniatis et al., Molecular Cloning, Cold Spring Harbor (1982).

DNA-Synthese

Die für die Herstellung der Aprotinin-Varianten benötigten DNA-Blöcke bzw. DNA-Primer (für gezielte Mutagenese) wurden mit dem DNA-Synthesizer 380 A der Fa. Applied Biosystems hergestellt. Die Reinigung der entschützten DNA-Oligonukleotide erfolgte routinemäßig über denaturierende Polyacrylamid-Gelelektrophorese oder als Tritylderivate mittels Hochdruck-Flüssigkeitschromtatographie (HPLC).

Gezielte Mutagenese

Die gezielte Mutagenese von spezifischen Aminosäurecodons oder Genabschnitten erfolgt nach der Methode von Eckstein (J. W. Taylor, J. Ott und F. Eckstein, Nucl. Acids Res. 13, 8764 - 8785, 1985) unter Verwendung des Mutagenese-Kits der Fa. Amersham-Buchler (Best.-Nr. RPN. 2322).

DNA-Sequenzierung

Zur Kontrolle der DNA-Sequenzen von Gen- und Vektorkonstruktionen wurde einzelsträngige, in M13-Vektoren subklonierte DNA nach der Methode von Sanger sequenziert (F. Sanger et. al., PNAS 74, 5463 - 5467, 1977). Doppelsträngige DNA wurde nach der Methode von M. Hattori und Y. Sakahi (Anal. Biochem. 152, 232 - 238, 1986) sequenziert.

Transformation von S. cerevisiae

Es wurden 100 ml einer Hefezellsuspension des Stamms SC106 (MAT-alpha, hom3, ga12, his6, ura3; Stamm S2207A, Yeast Genetics Stock Center, University of California Berkeley, CA 94720, USA) mit einer Zellkonzentration von $2 \times 10^7$/ml abzentrifugiert; das Zellsediment wurde einmal mit 5 ml TE-Puffer (10 mM Tris x HCl, pH 7,5, 1 mM EDTA) und dann mit 5 ml LiA-Puffer (0,1 M Lithium-Acetat in TE-Puffer) gewaschen. Die Zellen wurden dann in 1 ml LiA-Puffer suspendiert und 1 Stunde bei 30°C inkubiert.

Zu 0,1 ml Zellsuspension wurden 10 $\mu$l der Plasmidlösung (1 - 5 $\mu$g DNA) und 15 $\mu$l einer Carrier-DNA (denaturierte DNA aus Heringssperma, 3 mg/ml) gegeben. Nach einer Inkubation für 30 min bei 30°C wurden 0,7 ml Polypropylenglykol (40 % Polypropylenglykol 3350 in LiA-Puffer) zugegeben und weitere 60 min bei 30°C inkubiert. Die Zellen wurden danach einem Hitzeschock unterworfen (5 min, 42°C) und anschließend 4 sec in einer Eppendorf Mikrofuge abzentrifugiert. Das Zellpellet wurde zweimal mit jeweils 0,5 ml TE-Puffer gewaschen; die Zellen wurden dann in 0,1 ml TE-Puffer suspendiert und auf Selektiv-Nährboden ausplattiert. Transformanten wurden nach 3 Tagen Inkubation bei 30°C erhalten.

Wachstum von Transformanten und Analyse von Sekretionsprodukten

Transformanten wurden in SD-Medium (0,67 % Yeast Nitrogen-Base ohne Aminosäuren, 2 % D-Glucose) supplementiert mit Threonin, Methionin und Histidin (jeweils 20 mg/Ltr.) bei 30°C kultiviert. Nach Erreichen einer ausreichenden Zelldichte (i. d. R. $5 \times 10^9$ Zellen/ml) wurden die Zellen abzentrifugiert und die Trypsin- oder Elastase-hemmende Aktivität im Kulturüberstand gemessen.

Fermentation von Hefe-Transformanten für die Expression von rek. Aprotinin-Varianten im 10-Ltr. Maßstab

es wurden folgende Medien verwendet:

| SD | Bacto Yeast Nitrogen Base | 6,7 g/Ltr. |
| | Glucose | 20,0 g/Ltr. |
| SD2 | Bacto Yeast Nitrogen Base | 6,7 g/Ltr. |
| | Glucose | 20,0 g/Ltr. |
| | $KH_2PO_4$ | 6,7 g/Ltr. |
| SC6 | Difco yeast extract | 20,0 g/Ltr. |
| | $KH_2PO_4$ | 1,4 g/Ltr. |
| | $(NH_4)_2SO_4$ | 2,0 g/Ltr. |
| | $MgSO_4$ x 7 $H_2O$ | 0,25 g/Ltr. |
| | Antifoam SAG 471 (Union Carbide) | 0,1 ml/Ltr. |

Die Komponenten wurden im entionisiertem Wasser gelöst, der pH-Wert wurde auf 5,5 eingestellt. Die Nährlösungen wurden für 20 min bei 121°C sterilisiert. Glucose wurde in 1/5 des notwendigen Volumens an entionisiertem Wasser gelöst, getrennt sterilisiert und nach dem Abkühlen mit der Nährlösung vereinigt.

4

Stammkulturen:

Die Hefe-Transformanten wurden auf SD-Platten (Medium SD + 2 % Agar) bis zu 4 Wochen im Kühlschrank gehalten. Die Langzeitlagerung erfolgt in flüssigem Stickstoff.

Vorkulturen:

Vorkulturen wurden in Nährlösung SD2 im 1-Ltr. Schüttelkolben (Füllvolumen: 100 ml) hergestellt. Die Kolben wurden mit einer Einzelkolonie von der SD-Stammplatte beimpft und für 2 - 3 Tage bei 28°C auf einer Schüttelmaschine bei 280 Upm inkubiert (Schüttelkreisdurchmesser: 2,5 oder 5,0 cm).

10-Ltr. Fermentation:

10-Ltr.-Fermenter wurden mit dem Zellsediment aus 1,0-Ltr.-Vorkultur suspendiert in etwa 200 ml Vorkulturmedium beimpft. Die Fermentationsbedingungen waren: Nährlösung SC6, 28°C, Rührerdrehzahl 600 Upm, Belüftungsrate 0,5 vvm, pH-Kontrolle mit 2,5 N NaOH und 2,5 N $H_2SO_4$.

Fütterung:

Die Kulturen wurden kontinuierlich mit Glucose und einmal täglich mit Difco-Hefeextrakt gefüttert.

Glucose-Lösung:

500 g Glucose in einem Gesamtvolumen von 1000 ml; Beginn der Fütterung 4 Std. nach Beimpfung mit einer Rate von 0,02 ml/Ltr. x min, nach 10 - 20 Std. Erhöhung auf 0,1 ml/Ltr. x min.
Die Zufütterungsrate von Glucose wurde so gewählt, daß der Respirationsquotient nicht wesentlich über 1 stieg.
Difco-Hefeextrakt: Es wurde einmal täglich Hefeextrakt in einer Menge von 5 g/Ltr. zugegeben. Der Hefeextrakt wurde als Suspension in entionisiertem Wasser hergestellt.
Glucose- und Hefeextraktlösungen wurden bei 121°C für 20 min. sterilisiert.
Nach einer Fermentationsdauer von 96 Std. wurde unter den angegebenen Bedingungen ein Zelltrockengewicht von ca. 30 g/Ltr. erzielt. Die erzielte Produktausbeute lag bei 6 g/Ltr.

Polyacrylamid-Gelelektrophorese

Proteine wurden üblicherweise mit SDS-Polyacrylamid-Gelelektrophorese (Laemmli, Nature 277, 680, 1970) und Färbung mit Coomassie Brilliant Blau nachgewiesen.

Aminosäure-Analyse

Etwa 1 nMol Protein wurde in Gegenwart von 200 $\mu$l 6M HCl, 0,05 % $\beta$-Mercaptoethanol unter Vakuum bei 110°C 22 h inkubiert. Die Hydrolysate wurden getrocknet, in 150 $\mu$l 0,2 M Natrium-Citratpuffer pH 2,2 gelöst und filtriert. Die Aminosäureanalyse wurde an einem Biotronic LC 5000 Aminosäureanalysator mit Fluoreszenz-Detektor und Shimadzu C-R2AX Integrator durchgeführt. Die Aminosäuren wurden nach Reaktion mit o-Phthaldialdehyd entsprechend der Literatur quantifiziert (Benson & Hare, Proc. Natl. Acad. Sci, USA 72, 619, 1975).

Aminosäure-Sequenzierung

1 - 2 nMol Protein wurde nach Lösen in 30 $\mu$l Trifluoressigsäure auf Polybren-behandelte Glasfaserfilter aufgebracht und im Gasphasensequenator (Applied Biosystems) nach Hewick et al. (J. Biol. Chem. 256, 7990, 1981) sequenziert. Phenylthiohydantoin-Derivate wurden mit Hilfe einer Cyano-HPLC-Säule (DuPont) wie bei Beyreuther et al., Modern Methods in Protein Chemistry, 303 - 325, Walter de Gruyter, Berlin (1983) beschrieben mit Hilfe eines Waters HPLC-Systems separiert und analysiert.

Trypsin-Hemmtest

Die Trypsinaktivität wurde nach Geiger und Fritz Methods of Enzymatic Analysis, Vol. V 3rd. ed., Bergmeyer (ed), Verlag Chemie, Weinheim (1984), p. 121 mit Benzoyl-L-arginin-p-nitroanilid als Substrat bestimmt. Das freigesetzte p-Nitroanilin wurde spektrophometrisch bei 405 nm gemessen. Enzyn und Inhibitor wurden vor der Zugabe des Substrats 15 min vorinkubiert.

Elastase-Hemmtest

Humane Leukozyten-Elastase kam von Elastin Products Company, Inc. P.O. Box 147, Pacific , Miss, 63069/USA. Als Substrat wurde MeOSuc-Ala-Ala-Pro-Val-pNA (Bachem, Bubendorf/Schweiz) verwendet. Die Testbedingungen sind in Tabelle 3 angegeben. Allgemein wurden die Inhibitorproben nach Verdünnung mit Testpuffer und Zugabe von Substrat (gelöst in DMSO in einer Konzentration von 0,1 M und mit Puffer auf die Konzentration der Stammlösung gebracht) gestartet und die Freisetzung von p-Nitroanilin aus dem Substrat bei 405 nm kontinuierlich verfolgt. 100 %-Werte wurden in entsprechenden Tests ohne Inhibitoren bestimmt. Die Hemmung (in Prozent) wurde aus folgender Gleichung errechnet:

$$\% \text{ Inhibierung} = 100 \times \left(1 - \frac{\text{OD in Gegenwart von Inhibitor}}{\text{OD in Abwesenheit von Inhibitor}}\right)$$

$$\text{Puffer} \quad 0,2 \text{ M Tris/HCl, pH 8,0} + 0,1 \text{ \% Tween 80}$$

| Gesamtvolumen nach Substratzugabe | 0,65 ml |
|---|---|
| Enzymmenge/Test | 50 ng |
| Präinkubationszeit bei Raumtemperatur | 30 min |
| Substrat | MeO-Suc-Ala-Ala-Pro-Val-pNA |
| Stocklösung | 0,065 M |
| Menge/Test | 0,1 ml |
| Testtemperatur | $30^0$ C |

Tabelle 3: Bedingungen des Elastase-Hemmtests (Nakajima et al., J. Biol. Chem. 254, 4027, 1979).

Beispiel 1

Konstruktion und Klonierung der Gene für Val-15-Leu-17-, DesPro2-Val-15-Leu-17-, DesPro2-Arg-15-, DesPro2-Val-15-Leu-17-Arg-19- und DesPro2-Arg-15-Ala-17-Aprotinin

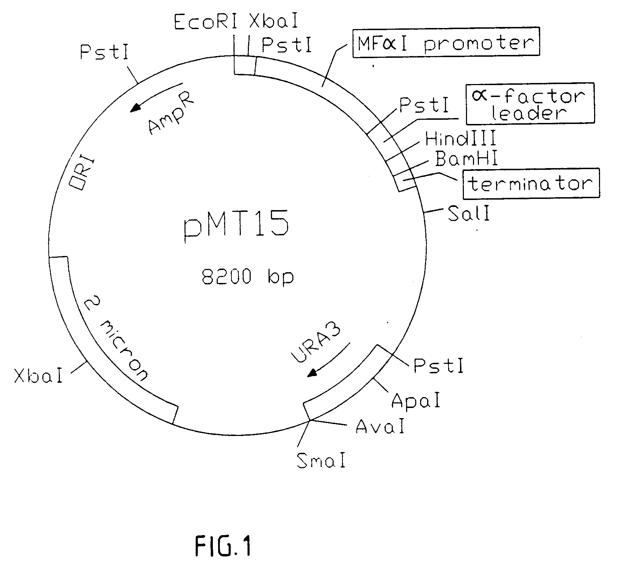

Für die Klonierung von Aprotinin-Muteinen in Hefe wurde ein Derivat des E. coli-Hefe-Shuttle-Vektors pMT 15 (Abb. 1) verwendet.

Der Vektor pMT 15 enthält das Ampicillin-Resistenz-Gen als selektiven Marker für E.coli und ein URA3-Genfragment für Hefe. Für die Replikation in E.coli und Hefe wurde der Col E1-Origin aus pBR 322 und ein DNA-Segment der B-Form des 2 µ-Plasmids aus Hefe verwendet. Für die Expression von heterologen Genen wurde der MAT 1-alpha-Promotor und die Kodierungsregion für die N-terminale Prä-Pro-Sequenz des alpha-Faktor-Vorläufer-Proteins in Form eines Eco RI - Hind III-Fragments aus dem Plasmid pCY 17 (Korjan und Herskowitz, Cell 30, 933, 1982) eingebaut.

Downstream von der alpha-Faktor-Prä-Pro-Sequenz enthält der Vektor pMT 15 ein Bam HI-Sal I-Fragment des URA3-Gens aus Hefe mit einer Transkriptions-Terminator-Funktion (Yarger et al., Mol. Cell. Biol. 8, 1095, 1986).

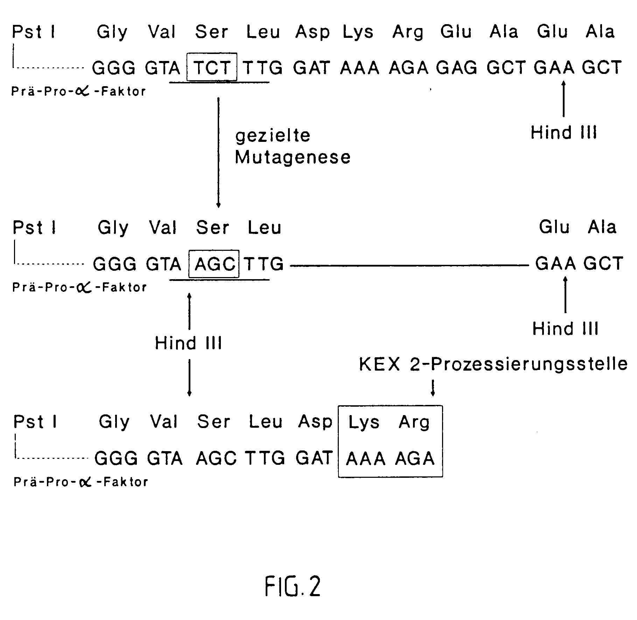

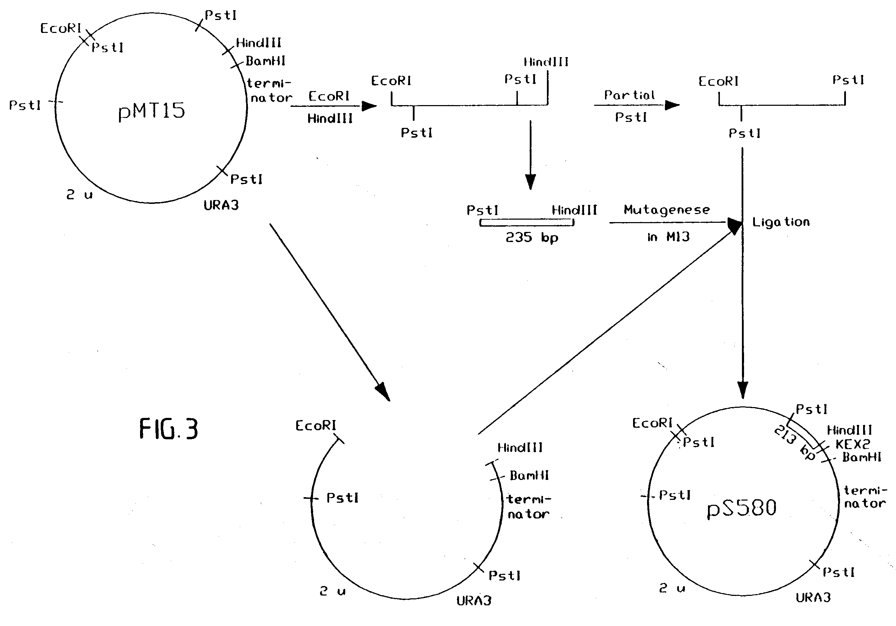

Das 235 bp PstI-Hind III-Fragment von pMT 15, das die kodierende Region der alpha-Faktor-Prä-Pro-Sequenz trägt, wurde in den Vektor M13 mp18 kloniert und einer gezielten Mutagenese unterworfen unter Verwendung des Oligonukleotid-Primers 5'-GAA-GAA GGG GTA TTG GAT AAA AGA-3'. Als Ergebnis der Mutagenese wurde das Serin-Kodon in Pos. 81 der alpha-Faktor-Prä-Pro-Sequenz von TCT zu AGC geändert, wodurch eine neue Hind III-Restriktionsschnittstelle erzeugt wurde (Abb. 2). Mit dem verkürzten 213 bp PstI-Hind III-Fragment wurde in pMT 15 das 235 bp Pst I-Hind III-Fragment ersetzt. Das so modifizierte Plasmid wurde mit pS 580 bezeichnet; es enthält als KEX2-Prozessierungsstelle die kodierende Sequenz für Lys-Arg statt Lys-Arg-Glu-Ala-Glu-Ala (Abb. 3).

Zur Fusion mit der alpha-Faktor-Prä-Pro-Sequenz in pS 580 wurde ein synthetisches Val-15-Leu-17-Aprotinin-Gen verwendet, das am 5'-Ende um die fünf letzten Codons der alpha-Faktor-Prä-Pro-Sequenz bis zur Hind-III-Schnittstelle in pS 580 verlängert wurde (Sequenzprotocoll am Ende der Beschreibung).

7

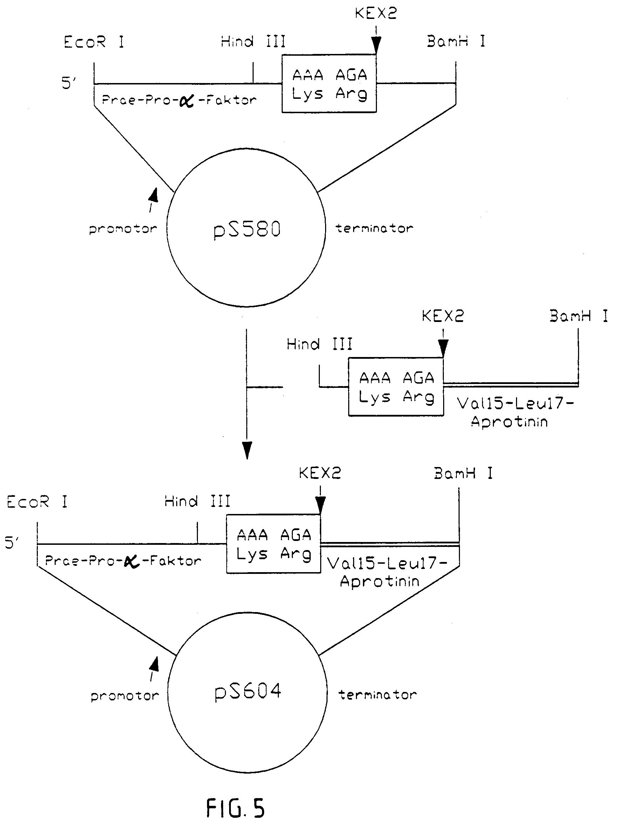

Das modifizierte Val-15-Leu-17-Aprotinin-Gen wurde in die geöffnete Hind III-BamHI-Schnittstelle von pS 580 eingebaut. Auf diese Weise wurde das 3′-Ende der alpha-Faktor-Prä-Pro-Sequenz mit der Lys-Arg-Prozessierungsstelle rekonstituiert und eine Leserasterfusion mit dem Val-15-Leu-17-Aprotinin-Gen erzeugt (Abb. 5).

Das pS 580-Derivat erhielt die Bezeichnung pS 604.

Zur Eliminierung der Aminosäure Prolin in Pos. 2 von Val-15-Leu-17-Aprotinin wurde das am 5′-Ende mit der Prä-Pro-Sequenz des alpha-Faktors fusionierte Aprotinin-Mutein als Hind III-Bam-HI-Kasettte aus pS 604 isoliert und in dem Mutagenese-Vektor M13-mp18 kloniert.

Zur Deletion des Prolin-Codons in Pos. 2 wurde im 1. Mutagenesezyklus der folgende synthetische Mutageneseprimer benutzt:

```
5'-AGC TTG GAT AAA AGA CGT GAC TTC TGC CTC GAG CCG CCG

TAC ACT GGG CC-3'.
```

Die Deletion des Prolin-Codons in Pos.2 des Val-15-Leu-17 Aprotinin-Gens wurde durch DNA-Sequenzierung verifiziert.

Für die Konstruktion des DesPro2-Arg-15-Aprotinin-Gens wurde das im M13-mp18-Mutagenese-Vektor klonierte DesPro2-Val-15-Leu-17-Aprotinin-Gen einem zweiten Mutagenese-Zyklus unterworfen.

Für den molekulargenetischen Austausch der Codons in Pos. 15 und Pos. 17 wurde der folgende Mutagenese-Primer verwendet:

```
5'-T GGG CCC TGC AGA GCT CGT ATC ATC CGT T-3'
              Arg       Arg
```

Der Austausch von Val-15 gegen Arg-15 und Leu-17 gegen Arg-17 wurde durch DNA-Sequenzierung bestätigt.

Anschließend wurden beide rek. Aprotinin-Muteine durch Einbau in die Hind III-Bam HI-Spaltstellen des Shuttle-Vektors pS 580 mit der alpha-Faktor-Prä-Pro-Sequenz fusioniert. Die pS 580 Derivate erhielten die Bezeichnung pS 707 (enthält DesPro2-Val-15-Leu-17-Aprotinin) und pA 202 (enthält DesPro2-Arg-15-Aprotinin).

Die Konstruktion und Klonierung der Muteine DesPro2-Val-15-Leu-17-Arg-19-Aprotinin (Vektor pS773) und DesPro2-Arg-15-Ala-17-Aprotinin (Vektor pA206) erfolgte in der oben beschriebenen Weise.

Beispiel 2

Konstruktion der Gene für Ala(-2)-Gln(-1)-Val-15-Leu-17-, Ala(-2)-Gln(-1)-Arg-15, Ala(-2)-Gln(-1)-Val-15-Leu-17-Arg-19- und Ala(-2)-Gln(-1)-Arg-15-Ala-17-Aprotinin

Für die gentechnische Addition des Dipeptids Ala-Gln an den N-Terminus der o.g. Aprotinin-Muteine wurden die in dem M13-Mutagenese-Vektor klonierten Gene für DesPro2-Val-15-Leu-17-Aprotinin und DesPro2-Arg-15-Aprotinin einem weiteren Mutagenese-Zyklus unter Verwendung des folgendes DNA-Primers unterworfen:

```
5'-AGC TTG GAT AAA AGA GCT CAA CGT CCG GAC TTC TGC C-3'
              Ala Gln
```

Die am 5′-Terminus modifizierte DNA-Sequenz der beiden Aprotinin-Muteine wurde durch DNA-Sequenzierung bestätigt.

Die Klonierung des Ala(-2)-Gln(-1)-Val-15-Leu-17-Aprotinin-Gens und Ala(-2)-Gln(-1)-Arg-15-Aprotinin-Gens in den Hefe Shuttle-Vektor pS 580 erfolgte analog zu den in Beispiel 1 beschriebenen Verfahren.

Die pS 580-Derivate mit den klonierten Aprotinin-Muteinen Ala(-2)-Gln(-1)-Val-15-Leu-17-Aprotinin und Ala(-2)-Gln-(-1)-Arg-15-Aprotinin erhielten die Bezeichnung pS 744 und pA 204.

Die Konstruktion und Klonierung der Muteine Ala(-2)-Gln(-1)-Val-15-Leu-17-Arg-19-Aprotinin (Vektor pS774) und Ala(-2)-Gln(-1)-Arg-15-Ala-17-Aprotinin (Vektor pA 207) erfolgte in der oben beschriebenen Weise.

Beispiel 3

Expression der rek. Aprotinin-Varianten in Hefe

Entsprechend der oben beschriebenen Methode wurde der Hefe-Stamm SC 106 mit dem Plasmid-Vektoren pS 604, pS 707, pS 744, pS 773, pS 774, pA 202, pA 204 und pA 207 transformiert.

Die URA $3^+$-Hefe-Transformanten wurden isoliert und unter Induktionsbedingungen (s. Methodenteil) kultiviert. Zur Ausbeutemessung wurden die Kulturüberstände im Fall der zu exprimierenden Aprotinin-Muteine mit Valin in Pos. 15 und Leucin in Pos. 17 auf Elastase-Hemmaktivität geprüft. Im Falle der zu exprimierenden Aprotinin-Muteine mit Arginin in Pos. 15 wurde der o.g. Trypsin-Hemmtest durchgeführt. Anschließend wurden die im 10-Ltr.-Fermenter sekretierten Expressionsprodukte der Aprotinin-Muteine aufgereinigt und charakterisiert.

Beispiel 4

Reinigung der rek. Aprotinin-Varianten

Fermentationsbrühen aus 10-Ltr.-Ansätzen wurden bei 9000 Upm abzentrifugiert (15 - 30 Minuten). Die Überstände wurden über verschiedene Filter (8 - 0,2 μm) filtriert, mit Wasser auf eine Leitfähigkeit von 7,5 mS verdünnt und mit Zitronensäure auf pH 3 eingestellt. Die so vorbehandeltem Proben wurden mit 100 - 200 ml S-Sepharose Fast Flow (Pharmacia) in 50 mM Natriumcitrat pH 3 versetzt und 30 - 60 Minuten gerührt. Anschließend wurde das Gel mit 1 - 5 Ltr. 50 mM Natriumcitrat pH 3, 50 mM TRIS HCl pH 9 und schließlich 20 mM HEPES pH 6 gewaschen. Das gewaschene Gel wurde in eine geeignete Säule überführt und am BIO-PILOT-System (Pharmacia) mit einem Gradienten zwischen 0 und 1 M Natriumchlorid in 20 mM HEPES pH 6 eluiert und fraktioniert. Nach Bestimmung der Hemmaktivität im Hemmtest mit humaner Leukozytenelastase bzw. Rindertrypsin wurden die entsprechenden Fraktionen gesammelt und im Rotationsverdampfer eingeengt.

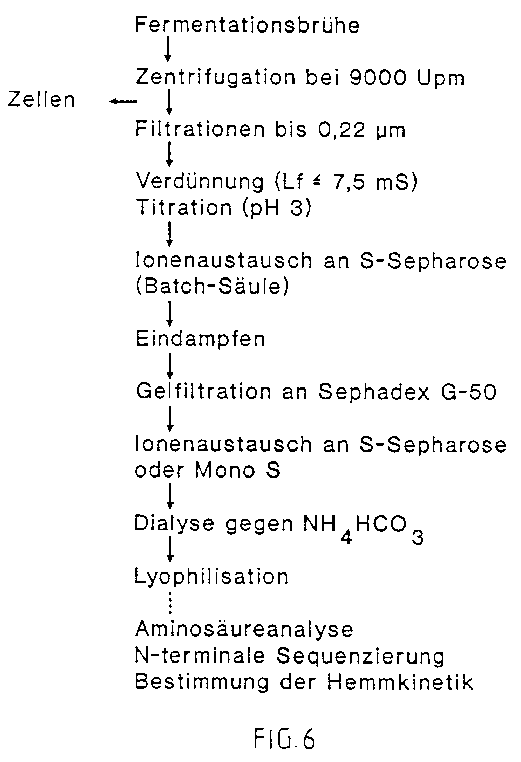

Dieses Material wurde weiter gereinigt durch Gelfiltration an Sephadex G-50 superfine (Pharmacia) und Chromatographie an S-Sepharose Fast Flow bzw. S-Sepharose HP oder Mono S (Pharmacia) in 20 mM HEPES pH 6. Von S-Sepharose wurde mit einem Gradienten zwischen 0 und 1 M NaCl eluiert. Fraktionen wurden durch Gelelektrophorese und entsprechenden Hemmtests überprüft. Fraktionen mit Hemmaktivität wurden gesammelt, gegen 0,1 M NH$_4$ HCO$_3$ dialysiert und gefriergetrocknet (Abb. 6).

Typischerweise wurden Ausbeuten von 20 - 40 % bezogen auf die im Fermentationsansatz vorhandene Menge an Inhibitoren erhalten.

Beispiel 5

Charakterisierung der nach Beispiel 4 erhaltenen Inhibitoren

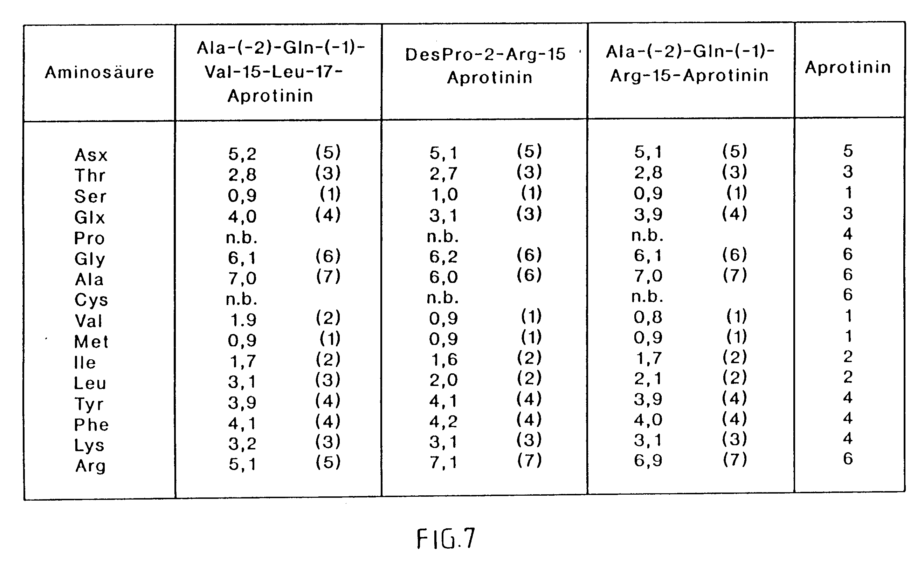

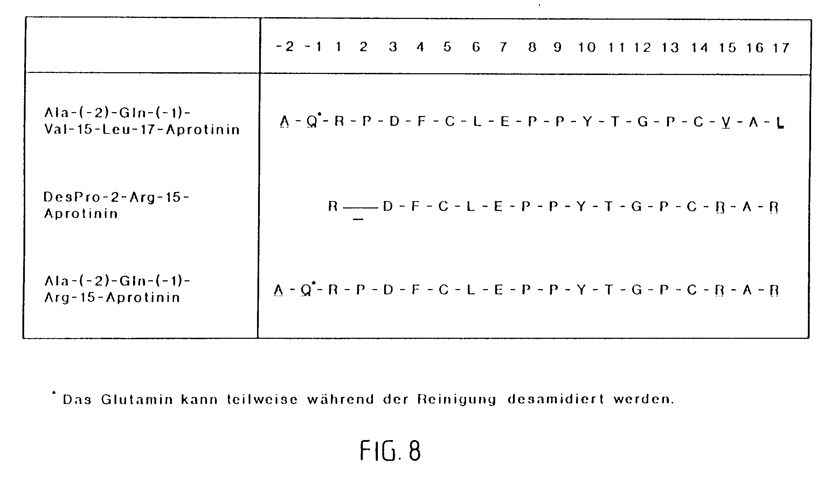

Die Lyophilisate wurden zunächst durch Aminosäureanalyse (Abb. 7) und N-terminale Sequenzierung (Applied Biosystems Sequenator) charakterisiert (Abb. 8).

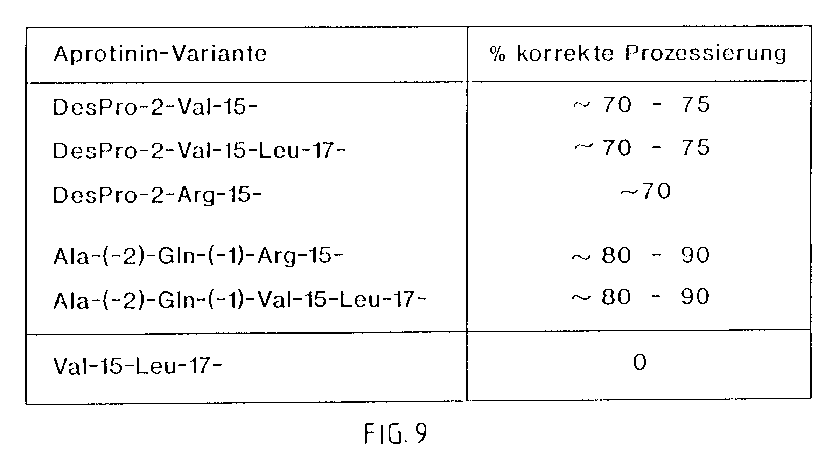

Im Falle des Val-15-Leu-17-Aprotinins wurde kein sekretiertes Material gefunden, das am N-Terminus dieser Aprotinin-Variante korrekt gespalten war (korrekte Prozessierung). Demgegenüber wurden die Aprotinin-Varianten, die entweder die Deletion in Pos. 2 oder aber die N-terminale Extension Ala(-2)-Gln(-1) aufweisen, zu 70 - 90 % mit dem korrekt prozessierten N-Terminus gefunden (Abb. 8 und 9).

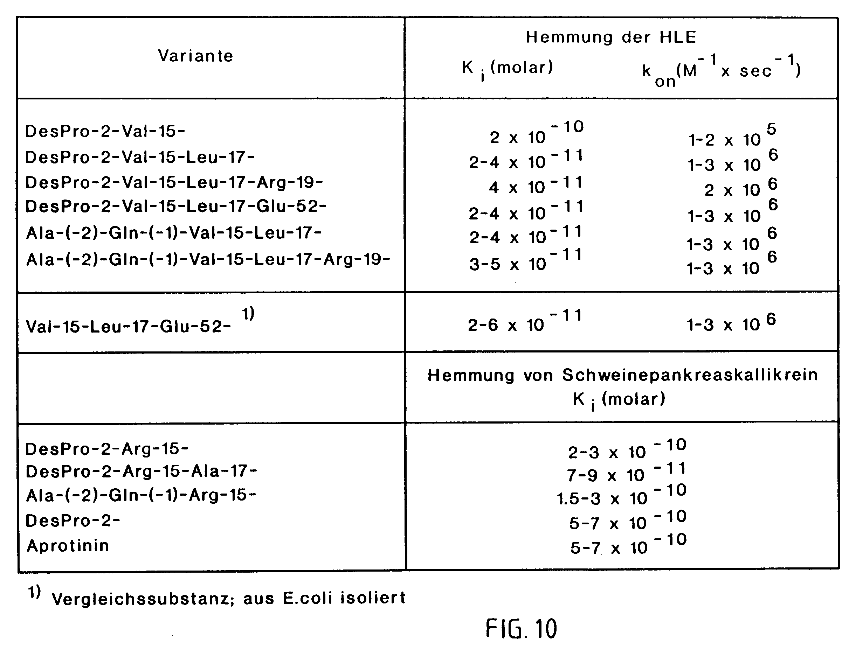

Zusätzlich wurden die Hemmkinetiken mit humaner Leukozytenelastase bzw. Schweine-Pankreaskallikrein bestimmt (Abb. 10). Dabei zeigte sich, daß die Veränderungen am N-Terminus keinerlei Einfluß auf die Hemmeigenschaften haben.

Legenden

Abb. 1    Restriktionskarte des E. coli-Hefe-Shuttle-Vektors pMT 15
Die für die Genexpression in Hefe essentiellen DNA-Signal-Sequenzen sind eingerahmt.

Abb. 2    Gentechnische Einführung einer neuen Hind-III-Schnittstelle in die Prä-Pro-alpha-Faktor-Sequenz
Die Hind-III-Erkennungssequenz wurde erzeugt durch Austausch des Kodons für Serin (TCT zu AGC).

Abb. 3    Konstruktionsschema des E. coli-Hefe-Shuttle-Vektors pS 580

Abb. 5    Konstruktionsschema des E. coli-Hefe-Shuttle-Vektors pS 604

Legenden - Fortsetzung

Abb. 6    Flußschema der Reinigung von Aprotinin-Varianten aus S.cerevisiae-Fermentationsüberständen

Abb. 7    Aminosäureanalysen einiger ausgewählter DesPro-2- und Ala-(-2)-Gln-(-1)-Varianten

Abb. 8    N-terminale Sequenzanalyse ausgewählter DesPro-2- und Ala-(-2)-Gln-(-1)-Varianten

Abb. 9    Prozessierung einiger DesPro-2- und Ala-(-2)-Gln-(-1)-Varianten durch die KEX2-Protease

Abb. 10   Inhibitorkonstanten der DesPro-2- und Ala-(-2)-Gln(-1)-Varianten

Sequenzprotokoll

Sequenz 1

EP 0 419 878 B1

Hind III

Xho I

```
   AAGCTTGGATAAAAGACGTCCGGACTTCTGCCTCGAGCCGCCGTACACTGGGCCCTGCGT
 1 --------+---------+---------+---------+---------+---------+ 60
   TTCGAACCTATTTTCTGCAGGCCTGAAGACGGAGCTCGGCGGCATGTGACCCGGGACGCA
   Ser Leu Asp Lys Arg Arg Pro Asp Phe Cys Leu Glu Pro Pro Tyr Thr Gly Pro Cys Val
```

α-F-Leader

KEX2

Stu I

```
   TGCTTTGATCATCCGTTACTTCTACAATGCAAAGGCAGGCCTGTGTCAGACCTTCGTATA
61 --------+---------+---------+---------+---------+---------+ 120
   ACGAAACTAGTAGGCAATGAAGATGTTACGTTTCCGTCCGGACACAGTCTGGAAGCATAT
   Ala Leu Ile Ile Arg Tyr Phe Tyr Asn Ala Lys Ala Gly Leu Cys Gln Thr Phe Val Tyr
```

Pst I                                         Sac II              Sph I

```
    CGGCGGCTGCAGAGCTAAGCGTAACAACTTCAAATCCGCGGAAGACTGCATGCGTACTTG
121 --------+---------+---------+---------+---------+---------+ 180
    GCCGCCGACGTCTCGATTCGCATTGTTGAAGTTTAGGCGCCTTCTGACGTACGCATGAAC
    Gly Gly Cys Arg Ala Lys Arg Asn Asn Phe Lys Ser Ala Glu Asp Cys Met Arg Thr Cys
```

BamH I

```
    CGGTGGTGCTTAGGCAAGCTCGGGATCC
181 --------+---------+-------- 208
    GCCACCACGAATCCGTTCGAGCCCTAGG
    Gly Gly Ala End
```

**Patentansprüche**
**Patentansprüche für folgende Vertragsstaaten : AT, BE, CH, DE, DK, FR, GB, GR, IT, LI, LU, NL, SE**

1. Aprotinin mit einer Deletion der Aminosäure Prolin in Pos. 2 oder einer Addition von Alanin-(-2)-Glutamin-(-1).

2. Aprotinin gemäß Anspruch 1 humanen oder tierischen Ursprungs.

3. Aprotinin gemäß den Ansprüchen 1 und 2 mit zusätzlichen Variationen in einer oder mehreren der Positionen 12, 13, 14, 15, 16, 17, 18, 19, 34, 36, 37, 38, 39, 41, 42 und 52.

4. Aprotinin-Varianten aus der Gruppe
   DesPro-2-Val-15-Leu-17,
   DesPro-2-Val-15-Leu-17-Arg19,
   Ala(-2)-Gln(-1)-Val-15-Leu-17,
   Ala(-2)-Gln(-1)-Val-15-Leu-17-Arg-19,
   DesPro-2-Arg-15,
   DesPro-2-Arg-15-Ala-17,
   Ala(-2)-Gln(-1)-Arg-15,
   Ala(-2)-Gln(-1)-Arg-15-Ala-17,

5. Arzneimittel enthaltend Aprotinin gemäß den Ansprüchen 1 bis 4.

6. Verfahren zur Herstellung von Aprotinin-Varianten gemäß Anspruch 1 bis 4, dadurch gekennzeichnet, daß mit einem geeigneten Vektor Hefen oder andere niedere Eukaryonten transformiert werden, die Transformanden kultiviert werden und die Aprotinin-Varianten aus der Kulturbrühe gereinigt werden.

7. Verfahren nach Anspruch 6, wobei der transformierte und kultivierte niedere Eukaryont S.cerevisiae ist.

**Patentansprüche für folgenden Vertragsstaat : ES**

1. Verfahren zur Herstellung von Aprotinin mit einer Deletion der Aminosäure Prolin in Pos. 2 oder einer Addition von Alanin-(-2)-Glutamin-(-1) dadurch gekennzeichnet, daß mit einem geeigneten Vektor Hefen oder andere niedere Eukaryonten transformiert werden, die Transformanden kultiviert werden und die Aprotinin-Varianten aus der Kulturbrühe gereinigt werden.

2. Verfahren gemäß Anspruch 1 zur Herstellung von Aprotinin humanen oder tierischen Ursprungs.

3. Verfahren gemäß den Ansprüchen 1 und 2 zur Herstellugn von Aprotinin mit zusätzlichen Variantionen in einer oder mehreren der Positionen 12, 13, 14, 15, 16, 17, 18, 19, 34, 36, 37, 38, 39, 41, 42 und 52.

4. Verfahren gemäß den Ansprüchen 1 - 3 zur Herstellung von Aprotinin-Varianten aus der Gruppe
   DesPro-2-Val-15-Leu-17,
   DesPro-2-Val-15-Leu-17-Arg19,
   Ala-(2)-Gln-(1)-Val-15-Leu-17,
   Ala(-2)-Gln(-1)-Val-15-Leu-17-Arg-19,
   DesPro-2-Arg-15,
   DesPro-2-Arg-15-Ala-17,
   Ala(-2)-Gln(-1)-Arg-15,
   Ala(-2)-Gln(-1)-Arg-15-Ala-17.

5. Verfahren gemäß den Ansprüchen 1 - 4, wobei der transformierte und kultivierte niedere Eukaryont S.cerevisiae ist.

**Claims**

**Claims for the following Contracting States : AT, BE, CH, DE DK, FR, GB, GR, IT, LI, LU, NL, SE**

1. Aprotinin with a deletion of the amino acid proline in pos. 2 or an addition of alanine-(-2)-glutamine-(-1).

2. Aprotinin according to Claim 1 of human or animal origin.

3. Aprotinin according to Claims 1 and 2 with additional variations in one or more of positions 12, 13, 14, 15, 16, 17, 18, 19, 34, 36, 37, 38, 39, 41, 42 and 52.

4. Aprotinin variants from the group
    DePro-2-Val-15-Leu-17,
    DePro-2-Val-15-Leu-17-Arg-19,
    Ala(-2)-Gln(-1)-Val-15-Leu-17,
    Ala(-2)-Gln(-1)-Val-15-Leu-17-Arg-19,
    DePro-2-Arg-15,
    DePro-2-Arg-15-Ala-17,
    Ala(-2)-Gln(-1)-Arg-15,
    Ala(-2)-Gln(-1)-Arg-15-Ala-17.

5. Medicament containing aprotinin according to Claims 1 to 4.

6. Process for the preparation of aprotinin variants according to Claims 1 to 4, characterized in that a suitable vector is used to transform yeasts or other lower eukaryotes, the transformants are cultivated, and the aprotinin variants are purified from the culture broth.

7. Process according to Claim 6, where the transformed and cultivated lower eukaryote is S. cerevisiae.

**Claims for the following Contracting State : ES**

1. Process for the preparation of aprotinin with a deletion of the amino acid proline in pos. 2 or an addition of alanine-(-2)-glutamine-(1), characterized in that a suitable vector is used to transform yeasts or other lower eukaryotes, the transformants are cultivated, and the aprotinin variants are purified from the culture broth.

2. Process according to Claim 1 for the preparation of aprotinin of human or animal origin.

3. Process according to Claims 1 and 2 for the preparation of aprotinin with additional variations in one or more of positions 12, 13, 14, 15, 16, 17, 18, 19, 34, 36, 37, 38, 39, 41, 42 and 52.

4. Process according to Claims 1-3 for the preparation of aprotinin variants from the group
    DePro-2-Val-15-Leu-17,
    DePro-2-Val-15-Leu-17-Arg-19,
    Ala(-2)-Gln(-1)-Val-15-Leu-17,
    Ala(-2)-Gln(-1)-Val-15-Leu-17-Arg-19,
    DePro-2-Arg-15,
    DePro-2-Arg-15-Ala-17,
    Ala(-2)-Gln(-1)-Arg-15,
    Ala(-2)-Gln(-1)-Arg-15-Ala-17.

5. Process according to Claims 1-4, where the transformed and cultivated lower eukaryote is S. cerevisiae.

**Revendications**
**Revendications pour les Etats contractants suivants : AT, BE, CH, DE, DK, FR, GB, GR, IT, LI, LU, NL, SE**

1. Aprotinine présentant une délétion de l'aminoacide proline en position 2 et une addition d'alanine-(-2)-glutamine-(-1).

2. Aprotinine suivant la revendication 1, d'origine humaine ou animale.

3. Aprotinine suivant les revendications 1 et 2, présentant des variations additionnelles dans une plusieurs des positions 12, 13, 14, 15, 16, 17, 18, 19, 34, 36, 37, 38, 39, 41, 42 et 52.

4. Variants de l'aprotinine, du groupe
   DesPro-2-Val-15-Leu-17,
   DesPro-2-Val-15-Leu-17-Arg19.
   Ala(-2)-Gln(-1)-Val-15-Leu-17,
   Ala(-2)-Gln(-1)-Val-15-Leu-17-Arg-19,
   DesPro-2-Arg-15,
   DesPro-2-Arg-15-Ala-17,
   Ala(-2)-Gln(-1)-Arg-15,
   Ala(-2)-Gln(-1)-Arg-15-Ala-17,

5. Médicament contenant de l'aprotinine suivant les revendications 1 à 4.

6. Procédé de production de variants de l'aprotinine suivant les revendications 1 à 4, caractérisé en ce qu'on transforme avec un vecteur approprié, des levures ou d'autres eucaryotes inférieurs, on cultive les transformants et on purifie les variants d'aprotinine extraits du bouillon de culture.

7. Procédé suivant la revendication 6, dans lequel l'eucaryote inférieur transformé et cultivé est S.cerevisiae.

**Revendications pour l'Etat contractant suivant : ES**

1. Procédé de production d'aprotinine présentant une délétion de l'aminoacide proline en position 2 et une addition d'alanine-(-2)-glutamine-(-1), caractérisé en ce qu'on transforme avec un vecteur approprié, des levures ou d'autres eucaryotes inférieurs, on cultive les transformants et on purifie les variants d'aprotinine extraits du bouillon de culture.

2. Procédé suivant la revendication 1, pour l'obtention d'aprotinine d'origine humaine ou animale.

3. Procédé suivant les revendications 1 et 2, pour l'obtention d'aprotinine présentant des variations additionnelles dans une ou plusieurs des positions 12, 13, 14, 15, 16, 17, 18, 19, 34, 36, 37, 38, 39, 41, 42 et 52.

4. Procédé suivant les revendications 1 à 3, pour l'obtention de variants d'aprotinine du groupe
   DesPro-2-Val-15-Leu-17,
   DesPro-2-Val-15-Leu-17-Arg19,
   Ala-(2)-Gln-(1)-Val-15-Leu-17,
   Ala(-2)-Gln(-1)-Val-15-Leu-17-Arg-19,
   DesPro-2-Arg-15,
   DesPro-2-Arg-15-Ala-17,
   Ala(-2)-Gln(-1)-Arg-15,
   Ala(-2)-Gln(-1)-Arg-15-Ala-17.

5. Procédé suivant les revendications 1 à 4, dans lequel l'eucaryote inférieur transformé et cultivé est S.cerevisiae.

FIG.1

Pst I      Gly   Val   Ser   Leu   Asp   Lys   Arg   Glu   Ala   Glu   Ala

Ɩ ·············· GGG GTA | TCT | TTG GAT AAA AGA GAG GCT GAA GCT

Prä-Pro-∝-Faktor

gezielte
Mutagenese

Hind III

Pst I      Gly   Val   Ser   Leu               Glu   Ala

Ɩ ············· GGG GTA | AGC | TTG ———————————— GAA GCT

Prä-Pro-∝-Faktor

Hind III

Hind III

KEX 2-Prozessierungsstelle

Pst I      Gly   Val   Ser   Leu   Asp | Lys   Arg

Ɩ ············· GGG GTA AGC TTG GAT | AAA AGA

Prä-Pro-∝-Faktor

FIG. 2

FIG. 3

EP 0 419 878 B1

FIG. 5

Fermentationsbrühe
↓
Zentrifugation bei 9000 Upm
Zellen ← ↓
Filtrationen bis 0,22 µm
↓
Verdünnung (Lf ≤ 7,5 mS)
Titration (pH 3)
↓
Ionenaustausch an S-Sepharose
(Batch-Säule)
↓
Eindampfen
↓
Gelfiltration an Sephadex G-50
↓
Ionenaustausch an S-Sepharose
oder Mono S
↓
Dialyse gegen $NH_4HCO_3$
↓
Lyophilisation
⋮
Aminosäureanalyse
N-terminale Sequenzierung
Bestimmung der Hemmkinetik

FIG. 6

| Aminosäure | Ala-(-2)-Gln-(-1)-Val-15-Leu-17-Aprotinin | | DesPro-2-Arg-15 Aprotinin | | Ala-(-2)-Gln-(-1)-Arg-15-Aprotinin | | Aprotinin |
|---|---|---|---|---|---|---|---|
| Asx | 5,2 | (5) | 5,1 | (5) | 5,1 | (5) | 5 |
| Thr | 2,8 | (3) | 2,7 | (3) | 2,8 | (3) | 3 |
| Ser | 0,9 | (1) | 1,0 | (1) | 0,9 | (1) | 1 |
| Glx | 4,0 | (4) | 3,1 | (3) | 3,9 | (4) | 3 |
| Pro | n.b. | | n.b. | | n.b. | | 4 |
| Gly | 6,1 | (6) | 6,2 | (6) | 6,1 | (6) | 6 |
| Ala | 7,0 | (7) | 6,0 | (6) | 7,0 | (7) | 6 |
| Cys | n.b. | | n.b. | | n.b. | | 6 |
| Val | 1.9 | (2) | 0,9 | (1) | 0,8 | (1) | 1 |
| Met | 0,9 | (1) | 0,9 | (1) | 0,9 | (1) | 1 |
| Ile | 1,7 | (2) | 1,6 | (2) | 1,7 | (2) | 2 |
| Leu | 3,1 | (3) | 2,0 | (2) | 2,1 | (2) | 2 |
| Tyr | 3,9 | (4) | 4,1 | (4) | 3,9 | (4) | 4 |
| Phe | 4,1 | (4) | 4,2 | (4) | 4,0 | (4) | 4 |
| Lys | 3,2 | (3) | 3,1 | (3) | 3,1 | (3) | 4 |
| Arg | 5,1 | (5) | 7,1 | (7) | 6,9 | (7) | 6 |

FIG.7

EP 0 419 878 B1

|  | -2 -1 1 2 3 4 5 6 7 8 9 10 11 12 13 14 15 16 17 |
|---|---|
| Ala-(-2)-Gln-(-1)-<br>Val-15-Leu-17-Aprotinin | A - Q*- R - P - D - F - C - L - E - P - P - Y - T - G - P - C - V - A - L |
| DesPro-2-Arg-15-<br>Aprotinin | R ——— D - F - C - L - E - P - P - Y - T - G - P - C - R - A - R |
| Ala-(-2)-Gln-(-1)-<br>Arg-15-Aprotinin | A - Q*- R - P - D - F - C - L - E - P - P - Y - T - G - P - C - R - A - R |

* Das Glutamin kann teilweise während der Reinigung desamidiert werden.

FIG. 8

| Aprotinin-Variante | % korrekte Prozessierung |
|---|---|
| DesPro-2-Val-15- | ~ 70 - 75 |
| DesPro-2-Val-15-Leu-17- | ~ 70 - 75 |
| DesPro-2-Arg-15- | ~70 |
| Ala-(-2)-Gln-(-1)-Arg-15- | ~ 80 - 90 |
| Ala-(-2)-Gln-(-1)-Val-15-Leu-17- | ~ 80 - 90 |
| Val-15-Leu-17- | 0 |

FIG. 9

| Variante | Hemmung der HLE | |
| --- | --- | --- |
| | $K_i$ (molar) | $k_{on}$ (M$^{-1}$ x sec$^{-1}$) |
| DesPro-2-Val-15- | $2 \times 10^{-10}$ | $1\text{-}2 \times 10^{5}$ |
| DesPro-2-Val-15-Leu-17- | $2\text{-}4 \times 10^{-11}$ | $1\text{-}3 \times 10^{6}$ |
| DesPro-2-Val-15-Leu-17-Arg-19- | $4 \times 10^{-11}$ | $2 \times 10^{6}$ |
| DesPro-2-Val-15-Leu-17-Glu-52- | $2\text{-}4 \times 10^{-11}$ | $1\text{-}3 \times 10^{6}$ |
| Ala-(-2)-Gln-(-1)-Val-15-Leu-17- | $2\text{-}4 \times 10^{-11}$ | $1\text{-}3 \times 10^{6}$ |
| Ala-(-2)-Gln-(-1)-Val-15-Leu-17-Arg-19- | $3\text{-}5 \times 10^{-11}$ | $1\text{-}3 \times 10^{6}$ |
| Val-15-Leu-17-Glu-52-[1] | $2\text{-}6 \times 10^{-11}$ | $1\text{-}3 \times 10^{6}$ |
| | Hemmung von Schweinepankreaskallikrein $K_i$ (molar) | |
| DesPro-2-Arg-15- | $2\text{-}3 \times 10^{-10}$ | |
| DesPro-2-Arg-15-Ala-17- | $7\text{-}9 \times 10^{-11}$ | |
| Ala-(-2)-Gln-(-1)-Arg-15- | $1.5\text{-}3 \times 10^{-10}$ | |
| DesPro-2- | $5\text{-}7 \times 10^{-10}$ | |
| Aprotinin | $5\text{-}7 \times 10^{-10}$ | |

[1] Vergleichssubstanz; aus E.coli isoliert

FIG. 10